# EUROPÄISCHE PATENTSCHRIFT

(11) **EP 0 448 093 B1**
(45) Veröffentlichungstag und Bekanntmachung des Hinweises auf die Patenterteilung: **06.03.1996**
(21) Anmeldenummer: 91104422.0
(22) Anmeldetag: 21.03.1991
(51) Int. Cl.: C12N 15/15, C12P 21/02

(54) **Sekretion von Hirudinderivaten**
Secretion of hirudin derivatives
Sécrétion des dérivés de hirudine

(30) Priorität: 22.03.1990 DE 4009268
(43) Veröffentlichungstag der Anmeldung: 25.09.1991
(73) Patentinhaber: Consortium für elektrochemische Industrie GmbH, D-81379 München (DE)
(72) Erfinder: Schmid, Gerhard, Dr., W-8000 München 60 (DE); Habermann, Paul, Dr., W-6239 Eppstein (Taunus) (DE)

(56) Entgegenhaltungen:
- EP-A- 0 338 410
- EP-A- 0 352 228
- EP-A- 0 356 335
- FEBS LETTERS, Band 202, Nr. 2, Juni 1986, Seiten 373-377; J. DODT et al.
- APPLIED MICROBIOLOGY AND BIOTECHNOLOGY, Band 34, Nr. 2, November 1990, Seiten 203-207, Springer-Verlag; E. BENDER et al.

## Beschreibung

Die Erfindung betrifft ein Verfahren zur Gewinnung von Hirudinderivaten aus E. coli-Sekretormutanten, sowie ein Hirudinderivat mit der N-terminalen Aminosäuresequenz Ala-Thr-Tyr-Thr-Asp.

Hirudin ist ein Polypeptid mit 65 Aminosäuren und wurde ursprünglich aus dem Blutegel Hirudo medicinalis isoliert. Es wirkt als hochspezifischer Inhibitor für Thrombin, indem es mit Thrombin stabile Komplexe bildet und besitzt daher viele therapeutische Anwendungsmöglichkeiten, insbesondere zur Antikoagulations-Therapie (F. Markquardt, Biomed. Biochim. Acta 44 (1985), 1007-1013).

Nach Veröffentlichung der vollständigen Aminosäuresequenz von Hirudin (J. Dodt et al., FEBS LETTERS 165 (2), (1984), 180-184) war die Voraussetzung für die Herstellung von Hirudin durch rekombinante DNA-Techniken und Expression in Mikroorganismen gegeben.

Die EP-A-0 158 564 (Transgene) offenbart Klonierungsvektoren zur Expression von Hirudin oder Hirudin-Analoga in einer Wirtszelle, insbesondere einer bakteriellen Zelle. Das für Hirudin kodierende Gen wird dabei durch cDNA-Synthese, ausgehend von mRNA aus dem Blutegel Hirudo medicinalis gewonnen. Insbesondere wird ein Hirudin-Derivat mit der N-terminalen Sequenz Ile-Thr-Tyr-Thr-Asp beschrieben, sowie Verfahren zu seiner Gewinnung.

In der EP-A-0 168 342 (Ciba Geigy) werden DNA-Sequenzen offenbart, welche für die natürliche Aminosäuresequenz von Hirudin kodieren, wobei die N-terminale Aminosäuresequenz Val-Val-Tyr-Thr-Asp ist. Die Expression von Hirudin erfolgt in den Mikroorganismen E. coli und Saccharomyces cerevisiae intrazellulär.

Die EP-A-0 171 024 (Hoechst AG) offenbart ein Verfahren zur gentechnologischen Herstellung von Polypeptiden mit Hirudin-Aktivität, insbesondere in E. coli-Zellen, wobei die Zellen aufgeschlossen werden und aus dem Zellextrakt das Polypeptid mit Hirudin-Aktivität gewonnen wird. Ein gegebenenfalls vorhandener Fusionsprotein-Anteil kann durch proteolytische oder chemische Spaltung abgetrennt werden und das freigesetzte Hirudinmolekül aufgereinigt werden.

Der Gegenstand der DE-OS 34 45 571 (GEN-BIO-TEC) ist eine DNA-Sequenz, die für ein Protein mit der biologischen Aktivität von Hirudin kodiert, sowie ein Verfahren zur Gewinnung solcher Proteine aus E. coli-Zellen, die mit einem geeigneten rekombinanten Vektor transformiert sind, durch Lysis der Zellen.

Auch die Arbeit von Bergmann et al. (Biol. Chem. Hoppe Seyler 367 (1986), 731-740) beschreibt die Hirudin-Synthese in E. coli. Die Freisetzung des Hirudins aus den Zellen erfolgt durch Behandlung mit Toluol, wobei nur geringe Ausbeuten von etwa 500 ng/l A₅₇₈ Einheiten von Zellen erzielt werden.

Die EP-A-0 200 655 (Transgene), EP-A-0 252 854 (Transgene) und EP-A-0 225 633 (Ciba Geigy) offenbaren die Gewinnung durch Sekretion von Proteinen mit Hirudin-Aktivität aus einem eukaryontischen Wirtsorganismus, insbesondere Hefe, wobei die Expression auf einem Vektor erfolgt, der eine DNA-Sequenz enthält, die ein Signalpeptid stromaufwärts des Strukturgens enthält. Es wird die Sekretion von Hirudin-Derivaten mit der N-terminalen Sequenz Val-Val-Tyr-Thr-Asp, sowie der N-terminalen Sequenz Ile-Thr-Tyr-Thr-Asp in Hefe offenbart. Dabei werden Ausbeuten bis zu 100 mg/l angegeben.

In der DE-OS 39 00 626 (Hoechst AG) wird ein Hirudin-Derivat mit der N-terminalen Sequenz Leu-Thr-Tyr-Thr-Asp offenbart. Die Expression findet vorzugsweise in Hefe statt, wobei Promotor und Signalsequenz des Hefe-Pheromon-Gens MFα zur Expression und Sekretion des Hirudin-Derivats verwendet werden.

Alle die oben beschriebenen Verfahren zur Herstellung von Hirudin-Derivaten weisen jedoch Nachteile auf. So werden bei Verwendung von Hefe als Wirtsorganismus und Sekretion des Hirudins in das Kulturmedium relativ hohe Ausbeuten erhalten, jedoch ist die Kultivierung von Hefezellen langwieriger und anspruchsvoller als die von Bakterien, z.B. E. coli. In E. coli-Zellen hingegen ist jedoch die Ausheute relativ gering oder/und bei einem Aufschluß der Zellen sind komplizierte Isolations-Verfahren notwendig.

Gegenstand der vorliegenden Erfindung ist ein Hirudin derivat gemäß Anspruch 1.

Ein Weiterer Gegenstand der vorliegenden Erfindung ist ein Verfahren zur Gewinnung des genannten Hirudin-Derivates aus E. coli Stämmen, welche eine massive Proteinsekretion in das Kulturmedium aufweisen, dadurch gekennzeichnet, daß man
(1) einen rekombinanten Vektor konstruiert, auf dem sich das, für das genannte Hirudin-Derivat kodierende Genhinter einem DNA-Abschnitt befindet, der für ein bakterielles Signalpeptid kodiert,
(2) mit dem in Schritt (1) konstruierten rekombinanten Vektor einen E. coli Stamm, welcher eine massive Protein-Sekretion in das Kulturmedium aufweist transformiert,
(3) die transformierten Zellen in einem Medium kultiviert und
(4) das Hirudin-Derivat aus dem Medium gewinnt.

Unter dem Begriff Hirudin-Derivat gemäß vorliegender Erfindung sind von Hirudin abgeleitete Proteine zu verstehen, die als Thrombin-Inhibitoren wirken und eine spezifische Aktivität von mindestens 10.000 AT-U/mg (Antithrombin-Units) besitzen (Dodt et al., Biol. Chem. Hoppe Seyler 366 (1985), 379-385). Der Begriff Hirudin-Derivat beinhaltet auch Fusionsproteine mit einem bis zu etwa 50 Aminosäuren langen N-terminalen Fusionsanteil, der teilweise oder vollständig durch proteolytische oder chemische Spaltung entfernt werden kann, wobei als Spaltprodukt ein Hirudin-Derivat mit einer spezifischen Aktivität von mindestens 10.000 AT-U/mg entsteht.

Ein Vorteil des erfindungsgemäßen Verfahrens ist, daß durch die Sekretion des Hirudin-Derivats in das Zellmedium die Disulfidbrücken von Hirudin unter den oxidativen Bedingungen des Mediums richtig ausgebildet werden.

Ein Verfahren zur Herstellung von E. coli-Stämmen, welche eine massive Protein-Sekretion in das Kulturmedium aufweisen, ist in der EP-A-0 338 410 offenbart. Bei der Gewinnung von geeigneten E. coli-Sekretormutanten kann man insbesondere von E. coli DS410 (DSM 4513) oder E. coli BW7261 (DSM 5231) ausgehen. Der jeweilige E. coli-Stamm wird zunächst mit einem Plasmid transformiert, das eine, für ein sekretierbares Protein kodierende DNA-Sequenz enthält. Anschließend wird der transformierte E. coli-Stamm einer Mutagenese, z.B. durch Behandlung mit N-Methyl-N'-nitro-N-nitrosoguanidin unterworfen. Dann erfolgt eine Selektion auf geeignete Sekretormutanten-Stämme. Verwendet man als sekretierbares Protein z.B. α-Cyclodextringlykosyltransferase, so kann man Sekretormutanten durch eine Resistenz gegen die Zellwand-aktive Substanz D-Cycloserin erkennen. Weiterhin bewirkt die Sekretion der α-Cyclodextringlykosyltransferase (CGTase) eine Hydrolyse der Stärke im umgebenden Medium, was bei Verwendung eines Amylopektin-Azur-Mediums eine zusätzliche Selektionsmöglichkeit für Sekretormutanten ergibt.

Als rekombinanter Vektor für die vorliegende Erfindung sind Vektoren geeignet, die entweder in das E. coli-Genom integrieren können (z.B. Bakteriophage λ) oder in der transformierten E. coli-Zelle extrachromosomal vorliegen (z.B. Plasmide). Vorzugsweise verwendet man Plasmide.

Das Genkonstrukt auf dem rekombinanten Vektor, welches für ein Protein, bestehend aus Signalpeptid und dem Hirudin-Derivat kodiert, befindet sich vorzugsweise unter Kontrolle eines induzierbaren Promotors, besonders bevorzugt eines trp-lac-Fusionspromotors, der durch Lactose-oder IPTG-Zugabe (Isopropyl-β-D-thiogalactosid) induzierbar ist. Auf dem Vektor soll sich weiterhin ein Selektionsmarker-Gen und gegebenenfalls ein lac-Repressor-Gen befinden.

Als bakterielle Signalsequenz, die eine Sekretion des Hirudin-Derivats ermöglicht, sind prinzipiell alle bekannten Signalpeptide geeignet, die eine Permeation der Membran von E. coli-Zellen erlauben. Vorzugsweise verwendet man daher auch Signalpeptide aus gram-negativen Bakterien (z.B. Signalpeptide folgender Proteine von E. coli:
äußeres Membranprotein OmpA (DiRienzo et al., 1978 Ann. Rev. Biochem. 47: 481-532)
alkalische Phosphatase PhoA (Inouye et al., 1982 J. Bacteriol. 149: 434-439)
LamB-Protein (Hedgpeth et al., 1980 Proc. Nat. Acad. Sci. USA 77: 2621-2625)
Maltose Bindeprotein MalE (Bedouelle H. et al., 1980 Nature 285: 78 - 81)). Besonders bevorzugt verwendet man das α-CGTase-Signalpeptid.

Ein für das erfindungsgemäße Verfahren geeigneter Vektor ist z.B. das Plasmid pCM705 (Fig. 1), das aus dem in der EP-A-0 383 410 offenbarten Plasmid pCM703 durch Entfernung eines ca. 1 kb langen NruI-Fragments erhältlich ist. Dieser Vektor enthält ein Ampicillin-Resistenzgen, das Gen für den Iac-Repressor und das CGTase-Gen mit 5'-Ende. Ein für ein Hirudin-Derivat kodierendes Gen wird in den Vektor pCM705 so integriert, daß intrazellulär ein Vorläufermolekül mit dem Signalpeptid der α-CGTase an seinem N-terminalen Ende entsteht. Das GenKonstrukt steht unter Kontrolle des tac-Promotors. Mit dem auf diese Weise erhaltenen Plasmid kann ein E. coli-Sekretor-Mutantenstamm transformiert werden.

Positiv transformierte Klone werden im Schüttelkolben oder im Fermenter kultiviert. Bei Erreichen einer optischen Dichte (OD₆₀₀ ) von etwa 1 erfolgt eine Induktion durch IPTG (Isopropyl-β-D-thiogalactosid) oder Lactose.

Mittels eines Thrombin-Inaktivierungstests (Griesbach et al., Thrombosis Research 37, (1985), 347-350) wird dann der Verlauf der Produktion des Hirudinderivats bestimmt. Die Akkumulation von Fusionsproteinen wird durch HPLC-Chromatographie (reversed phase) analysiert. Der Präanteil von Fusionsproteinen kann dann abgespalten und das entstehende aktive Hirudinderivat aufgereinigt werden.

Folgende Beispiele sollen die Erfindung zusammen mit den Figuren 1 bis 5 näher erläutern.

Es zeigen:
- Fig. 1: das Plasmid pCM705
- Fig. 2: die DNA-Sequenz des synthetischen Hirudin-Gens aus pK152,
- Fig. 3: die Sequenzen der Oligonukleotide HIR1, HIR2 und HIR3,
- Fig. 4: das Plasmid pCM7051 und
- Fig. 5: das Plasmid pCM7053.

### Beispiel 1

### Konstruktion des Sekretionsvektors

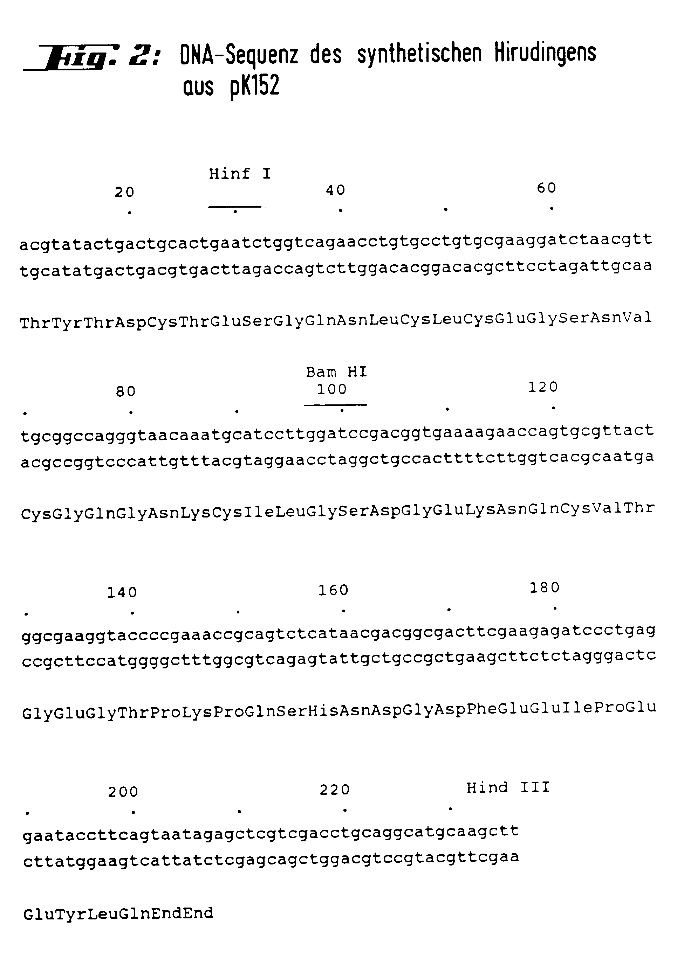

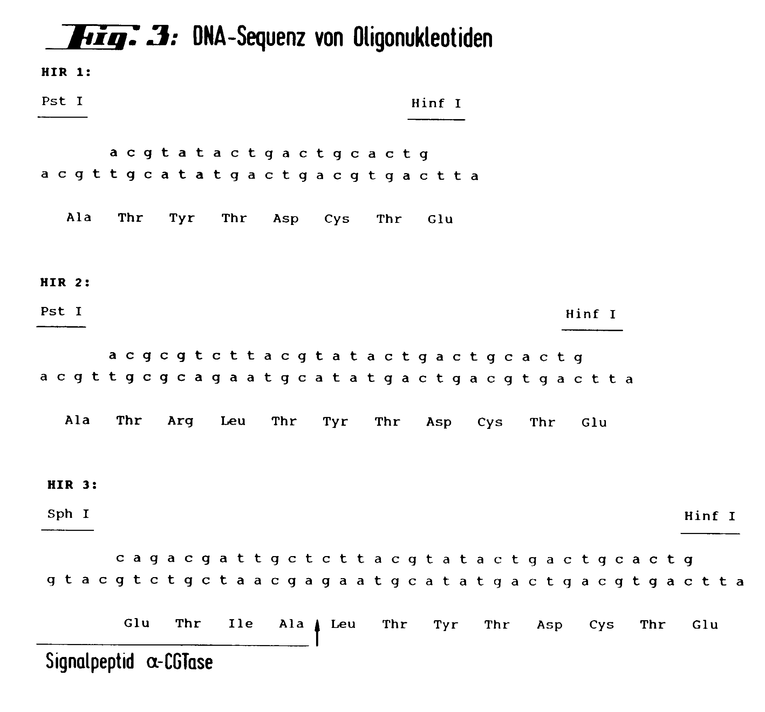

Das Plasmid pK152 trägt ein synthetisches Hirudingen, dessen Sequenz in der EP-A0171 024 aufgeführt ist. Ausgehend von diesem Plasmid wurde ein etwa 200 bp großes Hinf I - Hind III DNA-Fragment über Agarose-Gelelektrophorese isoliert, das den größten Teil der DNA-Sequenz, die für Hirudin kodiert, beinhaltet (Fig. 2). Die fehlende 5'-terminale Sequenz wird durch ein neusynthetisiertes Oligonukleotid (HIR 1) regeneriert. Die kodierende Sequenz des Oligonukleotids ist in Fig. 3 gezeigt. Durch Fusion der Hinf I-Enden ergibt sich ein Hirudin-Derivat mit der N-terminalen Sequenz Ala-Thr-Tyr-Thr-Asp.

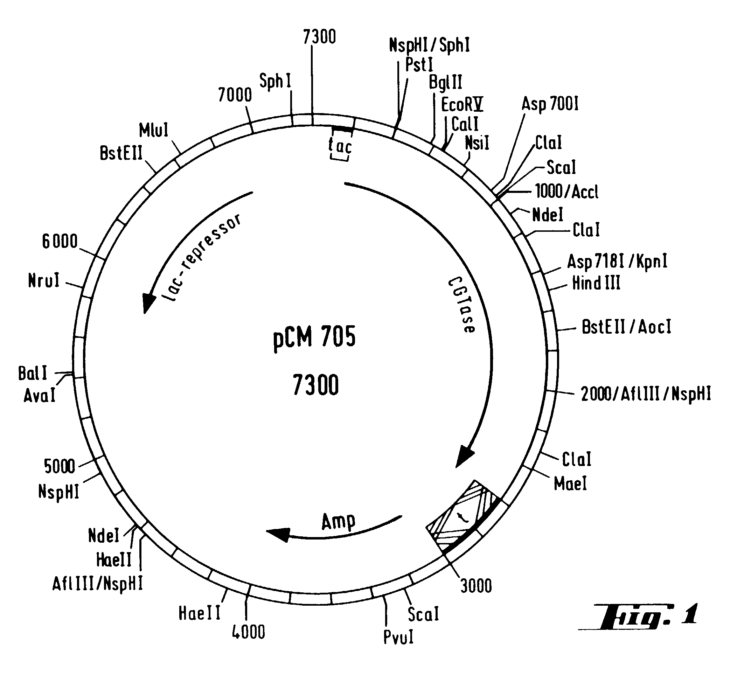

Das Plasmid pCM705 (Fig. 1) wird mit PstI und Hind III gespalten. Die beiden Schnittstellen liegen im kodierenden Bereich für das Gen der CGTase, wodurch ein etwa 1 kb großes DNA-Stück ausgeschnitten wird. PstI spaltet exakt in dem Bereich, der für die Signalpeptidase-Schnittstelle kodiert.

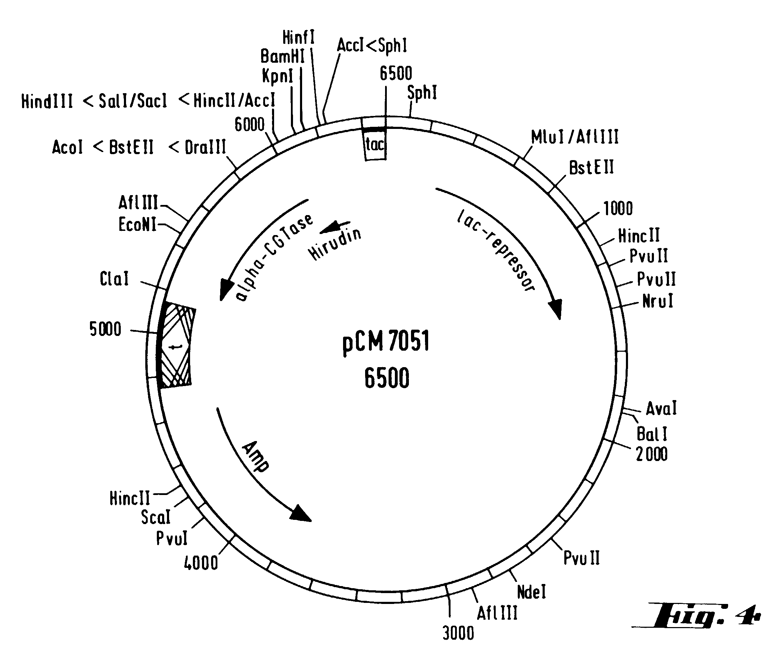

Die Fragmente pCM705 PstI- Hind III 6,3 kb, pK152 Hinf I - Hind III 0,2 kb und das Oligonukleotid HIR 1 werden miteinander ligiert, wodurch das Plasmid pCM7051 entsteht (Fig. 4). Mit dem Ligationsansatz wird der E. coli-Stamm HB101 (DSM 1607) transformiert. Kolonien, die auf selektivem Medium mit Amylopektin Azur (gefärbtes Amylopektin) keine Stärkeabbauhöfe und somit keine α-CGTase-Expression zeigen, werden isoliert und bis zur Einheitlichkeit gereinigt. Von mehreren gereinigten Klonen wird Plasmid-DNA isoliert und durch eine Restriktionsanalyse charakterisiert. Von zwei Plasmiden, die ein Hirudin-Insert tragen, wird eine Sequenzanalyse der Fusionsregionen durchgeführt.

Plasmid-DNA, die eine korrekte Hirudin-Genkonstruktion trägt, wird mit Nru I und Nde I gespalten und über Agarosegelelektrophorese wird ein 5,18 kb großes Fragment isoliert.

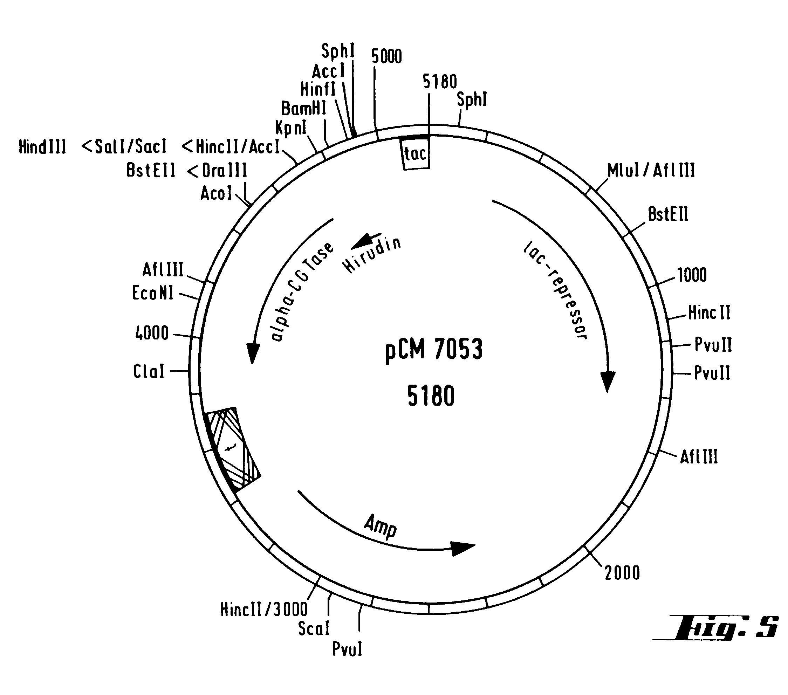

Nach Auffüllen der durch Nde I Spaltung überhängenden Sequenz mit Klenow-Enzym wird das Fragment durch Ligation zirkularisiert. Das entstandene Plasmid wird als pCM7053 bezeichnet (Fig. 5).

Mit diesem Plasmid pCM7053 wird die Sekretormutante E. coli WCM100 transformiert, die auf die in der EP-A-0 338 410 beschriebenen Methode erhalten wurde.

### Beispiel 2

### Test auf Sekretion von Hirudin in Schüttelkolben-Versuchen

10 ml LB-Medium mit 100 µg/ml Ampicillin wurde mit einer frischen Übernachtkultur von WCM100 pCM7053 auf die optische Dichte OD₄₂₀ = 0,1 beimpft. Die Kultur wird bei 30°C geschüttelt. Sobald die optische Dichte OD₄₂₀ = 1,0 erreicht, wird der Induktor Lactose auf eine Endkonzentration von 1 % zugegeben. Nach 48 Stunden werden von der Kultur Proben entnommen, die Zellen abzentrifugiert und die Hirudin-Konzentration im Überstand bestimmt. Die Bestimmung erfolgt mittels eines Thrombin Inaktivierungstests. Es wurden Ausbeuten bis zu 4000 AT-U/ml (Antithrombin Units) bestimmt (≙ 250 mg/l).

### Beispiel 3

### Hirudinproduktion im 10 l Fermenter

7 l Minimalmedium mit 100 µg/ml Ampicillin werden mit einer frischen Übernachtkultur von WCM100 pCM7053 auf die optische Dichte OD₆₀₀ = 0,1 angeimpft.

Die Fermentationsbedingungen sind

| | |
|---|---|
| Temperatur: | 30°C |
| Rührgeschwindigkeit: | 450 bis 950 rpm |
| Belüftung: | 0,5 bis 1,5 Vvm |
| pH-Wert: | 7,0 ± 0,1 |

Bei Erreichen der optischen Dichte OD₆₀₀ = 1,0 werden 0,5 mM IPTG (Isopropyl-β-D-Thiogalactosid) zugegeben.

40 Stunden nach Zugabe von IPTG konnten 36.000 AT-U/ml im Überstand bestimmt werden (≙ 2,25 g/l).

### Beispiel 4

### Sekretion von Hirudin mit der N-terminalen Sequenz Ala-Thr-Arg-Leu-Thr-Tyr-Thr-Asp

Verfährt man analog dem Beispiel 1, verwendet jedoch statt dem Oligonukleotid HIR 1 das Oligonukleotid HIR 2 (Fig. 3), so erhält man ein Hirudinfusionsprotein nach Abspaltung des Signalpeptides mit der N-terminalen Sequenz Ala-Thr-Arg-Leu-Thr-Tyr-Thr-Asp. Die Anhäufung dieses Fusionsproteins im Überstand wird durch HPLC-Analyse unter Anwendung von "Reversed Phase" Bedingungen (C₁₈-Chromatographiesäule) bestimmt. Die Fermentation des Stammes WCM100 mit diesem Genkonstrukt erbrachte eine Ausbeute von 25 mg/l Fusionsprotein.

Durch Trypsin-Spaltung kann aktives Hirudin mit der N-terminalen Sequenz Leu-Thr-Tyr-Thr-Asp erzielt werden.

### Beispiel 5

### Sekretion von Hirudin mit der Sekretormutante WCM88

Die Sekretormutante WCM88, die ebenfalls auf die in der EP-A-0 338 410 beschriebene Weise erhalten wurde, wird mit dem Plasmid pCM7053 transformiert (siehe Beispiel 1). Die Produktion von Hirudin durch Sekretion ins Kulturmedium wird in Schüttelkolben-Versuchen und Fermentationen getestet.
a) Schüttelkolbenversuche
   Analog Beispiel 2 wird der Stamm WCM88 pCM7053 kultiviert. Nach 48 Stunden wird im Überstand der Kultur die Hirudin-Konzentration bestimmt. Ausbeuten von bis zu 1 800 AT-U/ml wurden erzielt (≙ 110 mg/ l).
b) Produktion im 10 l Fermenter
   Die Anzucht des Stammes erfolgt wie in Beispiel 3 beschrieben. 45 Stunden nach Zugabe von IPTG konnten 21 000 AT-U/ml im Überstand festgestellt werden (≙ 1,3 g/l).

### Beispiel 6

### Konstruktion eines Sekretionsvektors mit Tetracyclinresistenzgen

Das 1,1 kb NruI-Fragment des Plasmids pBR322 (F. Bolivar et al., Gene 2, 95-113 (1977)) wurde isoliert und mit dem durch NruI-Spaltung linearisierten Plasmid PCM7051 (siehe Fig. 4) ligiert. Mit der Ligationsmischung wurde E. coli HB101 transformiert. Transformanten wurden aufgrund ihrer Tetracyclinresistenz selektiert. Die Plasmid-DNA eines selektierten Klons wurde reisoliert und mit NdeI und AvaI gespalten. Die DNA-Fragmente wurden in der Agarosegel-Elektrophorese aufgetrennt. Das größere Fragment wurde isoliert und die überhängenden Einzelstrangenden wurden mit dem Klenow-Enzym aufgefüllt und dann ligiert.

Das resultierende Plasmid war pCMT203.

### Beispiel 7

### Sekretion von Hirudin unter Verwendung des Sekretionsvektors pCMT203

Die Sekretormutante WCM100 (siehe Beispiel 1) wurde mit dem Plasmid pCMT203 transformiert. Der resultierende Stamm wurde in einem 10 l Fermenter kultiviert. 45 h nach Zugabe von IPTG konnten 42000 AT-U/ml Hirudin bestimmt werden (≙ 2,63 g/l).

## Patentansprüche (Patentansprüche für folgende(n) Vertragsstaat(en): AT, BE, CH, DE, DK, FR, GB, IT, LI, LU, NL, SE)

1. Hirudinderivat, dadurch gekennzeichnet, daß es die N-terminale Sequenz A-Thr-Tyr-Thr-Asp besitzt, worin A für Ala steht.

2. Rekombinante DNA, dadurch gekennzeichnet, daß sie für ein Hirudinderivat nach Anspruch 1 kodiert.

3. Rekombinanter Vektor, dadurch gekennzeichnet, daß er eine oder mehrere Kopien eines Genkonstruktes enthält, das für ein Protein, bestehend aus einem bakteriellen Signalpeptid und einem Hirudinderivat gemäß Anspruch 1 kodiert.

4. Verfahren zur Gewinnung von Hirudinderivaten gemäß Anspruch 1 aus E. coli-Stämmen, welche eine massive Protein-Sekretion in das Kulturmedium aufweisen, dadurch gekennzeichnet, daß man
(1) einen rekombinanten Vektor konstruiert, auf dem sich das für Hirudinderivat gemäß Anspruch 1 kodierende Gen direkt hinter einem DNA-Abschnitt befindet, der für ein bakterielles Signalpeptid kodiert,
(2) mit dem in Schritt (1) konstruierten rekombinanten Vektor einen E. coli-Stamm, welcher eine massive Protein-Sekretion in das Kulturmedium aufweist, transformiert,
(3) die transformierten Zellen in einem Medium kultiviert und
(4) das Hirudinderivat aus dem Medium gewinnt.

5. Verfahren nach Anspruch 4, dadurch gekennzeichnet, daß man als rekombinanten Vektor ein Plasmid verwendet.

6. Verfahren nach Anspruch 4 oder 5, dadurch gekennzeichnet, daß man einen rekombinanten Vektor konstruiert, auf dem sich die für das Hirudinderivat und das Signalpeptid kodierenden DNA-Sequenzen unter Kontrolle eines induzierbaren Promotors befinden.

7. Verfahren nach Anspruch 6, dadurch gekennzeichnet, daß man als induzierbaren Promotor einen trp-lac Fusionspromotor verwendet.

8. Verfahren nach einem der vorhergehenden Ansprüche, dadurch gekennzeichnet, daß man ein Signalpeptid aus gram-negativen Bakterien verwendet.

9. Verfahren nach Anspruch 8, dadurch gekennzeichnet, daß man das α-Cyclodextringlycosyltransferase-Signalpeptid verwendet.

## Patentansprüche (Patentansprüche für folgende(n) Vertragsstaat(en): ES, GR)

1. Verfahren zur Gewinnung von Hirudinderivaten gemäß Anspruch 1 aus E. coli-Stämmen, welche eine massive Protein-Sekretion in das Kulturmedium aufweisen, dadurch gekennzeichnet, daß man
(1) einen rekombinanten Vektor konstruiert, auf dem sich das für Hirudinderivat gemäß Anspruch 1 kodierende Gen direkt hinter einem DNA-Abschnitt befindet, der für ein bakterielles Signalpeptid kodiert,
(2) mit dem in Schritt (1) konstruierten rekombinanten Vektor einen E. coli-Stamm, welcher eine massive Protein-Sekretion in das Kulturmedium aufweist, transformiert,
(3) die transformierten Zellen in einem Medium kultiviert und
(4) das Hirudinderivat aus dem Medium gewinnt.

2. Verfahren nach Anspruch 1, **dadurch gekennzeichnet** , daß man als rekomninanten Vektor ein Plasmid verwendet.

3. Verfahren nach Anspruch 1 oder 2, **dadurch gekennzeichnet** , daß man einen rekombinanten Vektor konstruiert, auf dem sich die für das hirudinderivat und das Signalpeptid kodierenden DNA-Sequenzen unter Kontrolle eines induzierbaren Promotors befinden.

4. Verfahren nach Anspruch 3, **dadurch gekennzeichnet** , daß man als induzierbaren Promotor einen trp-lac Fusionspromotor verwendet.

5. Verfahren nach einem der vorhergehenden Ansprüche, **dadurch gekennzeichnet,** daß man ein Signalpeptid aus gram-negativen Bakterien verwendet.

6. Verfahren nach Anspruch 5, **dadurch gekennzeichnet** , daß man das α-Cyclodextringlycosyltransferase-Signalpeptid verwendet.

7. Verfahren nach einem der vorhergehenden Ansprüche, **dadurch gekennzeichnet,** daß man als E. coli-Sekretormutante einen Bakterienstamm verwendet, der durch Mutagenese und anschiießende Selektion auf Sekretionseigenschaften aus den E. coli-Stämmen DS410 (DSM 4513) oder BW 7261 (DSM 5231) erhalten wurde.

8. Rekombinante DNA, **dadurch gekennzeichnet** , daß sie für ein Hirudinderivat herstellbar nach einem der Ansprüche 1 bis 7 kodiert.

9. Rekombinanter Vektor, **dadurch gekennzeichnet** , daß er eine oder mehrere Kopien eines Genkonstruktes enthält, das für ein Protein, bestehend aus einem bakteriellen Signalpeptid und einem Hirudinderivat, mit der N-terminalen Sequenz Ala-Thr-Tyr-Thr-Asp kodiert.

10. Rekombinanter Vektor nach Anspruch 9, **dadurch gekennzeichnet**, daß das Signalpeptid aus gram-negativen Bakterien stammt.

11. Rekombinanter Vektor nach einem der Ansprüche 9 bis 10 dadurch **gekennzeichnet** , daß das Signalpeptid aus dem α-Cyclodextringlykosyltransferase-Gen stammt.

## Claims (Claims for the following Contracting State(s): AT, BE, CH, DE, DK, FR, GB, IT, LI, LU, NL, SE)

1. Hirudin derivative, characterised in that it has the N-terminal sequence A-Thr-Tyr-Thr-Asp in which A represents Ala.

2. Recombinant DNA, characterised in that it codes for a hirudin derivative according to Claim 1.

3. Recombinant vector, characterised in that it contains one or more copies of a gene construct which codes for a protein consisting of a bacterial signal peptide and of a hirudin derivative according to Claim 1.

4. Process for obtaining hirudin derivatives according to Claim 1 from E. coli strains which exhibit large-scale protein secretion into the culture medium, characterised in that
(1) a recombinant vector on which there is located the gene coding for [lacuna] hirudin derivative according to Claim 1 directly downstream of a DNA section which codes for a bacterial signal peptide is constructed,
(2) the recombinant vector constructed in step (1), is used to transform an E. coli strain which exhibits large-scale protein secretion into the culture medium,
(3) the transformed cells are cultivated in a medium and
(4) the hirudin derivative is obtained from the medium.

5. Process according to Claim 4, characterised in that a plasmid is used as recombinant vector.

6. Process according to Claim 4 or 5, characterised in that a recombinant vector on which the DNA sequences coding for the hirudin derivative and for the signal peptide are located under the control of an inducible promotor is constructed.

7. Process according to Claim 6, characterised in that a trp-lac fusion promotor is used as inducible promotor.

8. Process according to one of the preceding claims, characterised in that a signal peptide from Gram-negative bacteria is used.

9. Process according to Claim 8, characterised in that the α-cyclodextrin glycosoltransferase signal peptide is used.

## Claims (Claims for the following Contracting State(s): ES, GR)

1. Process for obtaining hirudin derivates according to Claim 1 [sic] from E. coli strains which exhibit a large-scale protein secretion into the culture medium, characterised in that
(1) a recombinant vector on which there is located the gene coding for [lacuna] hirudin derivative according to Claim 1 [sic] directly downstream of a DNA section which codes for a bacterial signal peptide is constructed,
(2) the recombinant vector constructed in step (1) is used to transform an E. coli strain which exhibits large-scale protein secretion into the culture medium,
(3) the transformed cells are cultivated in a medium and
(4) the hirudin derivative is obtained from the medium.

2. Process according to Claim 1, characterised in that a plasmid is used as recombinant vector.

3. Process according to Claim 1 or 2, characterised in that a recombinant vector on which the DNA sequences coding for the hirudin derivative and for the signal peptide are located under the control of an inducible promoter is constructed.

4. Process according to Claim 3, characterised in that a trp-lac fusion promotoer is used as inducible promoter.

5. Process according to one of the preceding claims, characterised in that a signal peptide from Gram-negative bacteria is used.

6. Process according to Claim 5, characterised in that the α-cyclodextrin glycosyltransferase signal peptide is used.

7. Process according to one of the preceding claims, characterised in that a bacterial strain which has been obtained by mutagenesis and subsequent selection for secretion properties from the E. coli strains DS410 (DSM 4513) or BW 7261 (DSM 5231) is used as E. coli secretor mutant.

8. Recombinant DNA, characterised in that it codes for a hirudin derivative which can be prepared according to one of Claims 1 to 7.

9. Recombinant vector, characterised in that it contains one or more copies of a gene construct which codes for a protein consisting of a bacterial signal peptide and of a hirudin derivative having the N-terminal sequence Ala-Thr-Tyr-Thr-Asp.

10. Recombinant vector according to Claim 9, characterised in that the signal peptide originates from Gram-negative bacteria.

11. Recombinant vector according to one of Claims 9 to 10, characterised in that the signal peptide originates from the α-cyclodextrin glycosyltransferase gene.

## Revendications (Revendications pour l'(les) Etat(s) contractant(s) suivant(s): AT, BE, CH, DE, DU, FR, IT, LI, LU, NL, SE)

1. Dérivé de la hirudine, caractérisé en ce qu'il possède la séquence N-terminale A-Thr-Tyr-Thr-Asp, où A est placé pour Ala.

2. ADN recombiné, caractérisé en ce qu'il code pour un dérivé de la hirudine suivant la revendication 1.

3. Vecteur recombiné, caractérisé en ce qu'il contient une ou plusieurs copies de la construction de gène, qui code pour une protéine constituée d'un peptide signal bactérien et d'un dérivé de la hirudine suivant la revendication 1.

4. Procédé d'obtention de dérivés de la hirudine suivant la revendication 1, à partir de souches de E. coli qui présentent une sécrétion massive de protéine dans le milieu de culture, caractérisé en ce que l'on :
(1) construit un vecteur recombiné dans lequel le gène codant pour le dérivé de la hirudine suivant la revendication 1, se trouve directement après un fragment d'ADN qui code pour un peptide signal bactérien,
(2) transforme une souche de E. coli avec le vecteur recombiné construit à l'étape (1), qui présente une sécrétion massive de protéine dans le milieu de culture,
(3) met en culture les cellules transformées dans un milieu et
(4) le dérivé de la hirudine est récolté dans le milieu.

5. Procédé suivant la revendication 4, caractérisé en ce que l'on utilise un plasmide comme vecteur recombiné.

6. Procédé suivant la revendication 4 ou 5, caractérisé en ce que l'on construit un vecteur recombiné dans lequel les séquences d'ADN codant pour le dérivé de la hirudine et le peptide signal se trouvent sous le contrôle d'un promoteur inductible.

7. Procédé suivant la revendication 6, caractérisé en ce que l'on utilise un promoteur de fusion trp-lac comme promoteur inductible.

8. Procédé suivant l'une quelconque des revendications précédentes, caractérisé en ce que l'on utilise un peptide signal de bactéries gram-négatives.

9. Procédé suivant la revendication 8, caractérisé en ce que l'on utilise le peptide signal de l'α-cyclodextrineglycosyltransférase.

## Revendications (Revendications pour l'(les) Etat(s) contractant(s) suivant(s): ES, GR)

1. Procédé d'obtention de dérivés de la hirudine suivant la revendication 1, à partir de souches de E. coli qui présentent une sécrétion massive de protéine dans le milieu de culture, caractérisé en ce que l'on :
(1) construit un vecteur recombiné dans lequel le gène codant pour le dérivé de la hirudine suivant la revendication 1, se trouve directement après un fragment d'ADN qui code pour un peptide signal bactérien,
(2) transforme une souche de E. coli avec le vecteur recombiné construit à l'étape (1), qui présente une sécrétion massive de protéine dans le milieu de culture,
(3) met en culture les cellules transformées dans un milieu et
(4) le dérivé de la hirudine est récolté dans le milieu.

2. Procédé suivant la revendication 1, caractérisé en ce que l'on utilise un plasmide comme vecteur recombiné.

3. Procédé suivant la revendication 1 ou 2, caractérisé en ce que l'on construit un vecteur recombiné dans lequel les séquences d'ADN codant pour le dérivé de la hirudine et le peptide signal se trouvent sous le contrôle d'un promoteur inductible.

4. Procédé suivant la revendication 3, caractérisé en ce que l'on utilise un promoteur de fusion trp-lac comme promoteur inductible.

5. Procédé suivant l'une quelconque des revendications précédentes, caractérisé en ce que l'on utilise un peptide signal de bactéries gram-négatives.

6. Procédé suivant la revendication 5, caractérisé en ce que l'on utilise le peptide signal de l'α-cyclodextrineglycosyltransférase.

7. Procédé suivant l'une quelconque des revendications précédentes, caractérisé en ce que l'on utilise une souche bactérienne comme mutant sécréteur de E. coli, qui est obtenue à partir des souches de E. coli DS410 (DSM 4513) ou BW 7261 (DSM 5231) par mutagenèse et ensuite, sélection sur les propriétés de sécrétion.

8. ADN recombiné, caractérisé en ce qu'il code pour un dérivé de la hirudine pouvant être préparé suivant l'une quelconque des revendications 1 à 7.

9. Vecteur recombiné, caractérisé en ce qu'il contient une ou plusieurs copies d'une construction de gène qui code pour une protéine constituée d'un peptide signal bactérien et d'un dérivé de la hirudine avec la séquence N-terminale Ala-Thr-Tyr-Thr-Asp.

10. Vecteur recombiné suivant la revendication 9, caractérisé en ce que le peptide signal provient de bactéries gram-négatives.

11. Vecteur recombiné suivant l'une quelconque des revendications 9 et 10, caractérisé en ce que le peptide signal provient du gène de l'α-cyclodextrine-glycosyltransférase.
